(19) Europäisches Patentamt
European Patent Office
Office européen des brevets

(11) Veröffentlichungsnummer: **0 358 988 A1**

(12) # EUROPÄISCHE PATENTANMELDUNG

(21) Anmeldenummer: **89115541.8**

(22) Anmeldetag: **23.08.89**

(51) Int. Cl.[5]: **C07C 43/13 , C07C 43/11 , C07C 69/708 , C07D 303/40 , C07C 41/26**

(30) Priorität: **01.09.88 DE 3829735**

(43) Veröffentlichungstag der Anmeldung: **21.03.90 Patentblatt 90/12**

(84) Benannte Vertragsstaaten: **ES GR**

(71) Anmelder: **Henkel Kommanditgesellschaft auf Aktien**
**Postfach 1100 Henkelstrasse 67**
**D-4000 Düsseldorf 1(DE)**

(72) Erfinder: **Stoll, Gerhard, Dr.**
**Danziger Strasse 69**
**D-4052 Korschenbroich 1(DE)**
Erfinder: **Höfer, Rainer, Dr.**
**Klever Strasse 31**
**D-4000 Düsseldorf 30(DE)**
Erfinder: **Döbrich, Peter**
**Schützenstrasse 9**
**D-4000 Düsseldorf(DE)**

(54) **Verfahren zur Herstellung von Fettalkoholderivaten.**

(57) Ein Verfahren zur Herstellung von Fettalkoholderivaten der allgemeinen Formel I

$R^1$(vic. OH, $OR^2$) - OH (I)

in der

$R^1$ einen vicinal disubstituierten Rest eines Fettalkohols mit 16 bis 22 Kohlenstoffatomen, der mindestens ein Strukturelement der allgemeinen Formel II

- CH(OH, - CH($OR^2$) - (II)

aufweist und

$R^2$ einen Alkylrest mit 1 bis 22 Kohlenstoffatomen, einen einbindigen Rest eines Polyols mit 2 bis 12 Kohlenstoffatomen und 2 bis 4 OH-Gruppen oder

eine Gruppe der allgemeinen Formel III

$$\begin{array}{c} | \\ R^3 \\ | \\ R^1(\text{vic. OH, } O) - OH \end{array} \qquad (III)$$

in der $R^3$ ein zweibindiger Rest eines Polyols mit 2 bis 12 Kohlenstoffatomen und 2 bis 4 OH-Gruppen, von denen mindestens 2 primäre OH-Gruppen sind, und $R^1$ wie oben definiert ist, bedeuten, wobei man einen vicinal hydroxy, alkoxy- bzw. hydroxy, hydroxyalkylenoxy-substituierten $C_{16}$-$C_{22}$-Fettsäureester mit Cu- und/oder Zn-haltigen Katalysatoren bei erhöhten Temperaturen und Druck hydriert, ergibt die gewünschten Verbindungen mit geringen Anteilen an Nebenprodukten.

EP 0 358 988 A1

## Verfahren zur Herstellung von Fettalkoholderivaten.

## Verfahren zur Herstellung von Fettalkoholderivaten.

Die Erfindung betrifft ein Verfahren zur Herstellung von Fettalkoholderivaten der allgemeinen Formel I

$R^1$(vic. OH, $OR^2$) - OH (I)

in der

$R^1$ einen vicinal disubstituierten Rest eines Fettalkohols mit 16 bis 22 Kohlenstoffatomen, der mindestens ein Strukturelement der allgemeinen Formel II

- CH(OH) - CH($OR^2$) - (II)

aufweist und

$R^2$ einen Alkylrest mit 1 bis 22 Kohlenstoffatomen, einen einbindigen Rest eines Polyols mit 2 bis 12 Kohlenstoffatomen und 2 bis 4 OH-Gruppen oder

eine Gruppe der allgemeinen Formel III

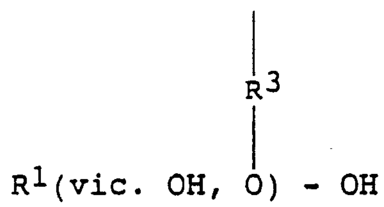

(III)

in der $R^3$ ein zweibindiger Rest eines Polyols mit 2 bis 12 Kohlenstoffatomen und 2 bis 4 OH-Gruppen, von denen mindestens 2 primäre OH-Gruppen sind, und $R^1$ wie oben definiert ist, bedeuten.

Die Herstellung von vicinal hydroxy, alkoxy- bzw. hydroxy, hydroxyalkylenoxy-substituierten $C_{16}$-$C_{22}$-Fettalkoholen, die verzweigte, nicht verseifbare Polyole darstellen, erfolgte bisher auf dem Wege über die Epoxidation der entsprechenden ungesättigten Fettalkohole mit anschließender Ringöffnung des Oxiran-Ringes mit einem ein- oder mehrwertigen Alkanol. Man erhielt auf diese Weise Ethergruppen enthaltende Polyole, deren OH-Funktionalität von der ursprünglichen Anzahl der Doppelbindungen, der Wertigkeit des zur Ringöffnung verwendeten Alkanols sowie dem Molverhältnis von Epoxyfettalkohol zu Alkanol bei der Ringöffnung abhängt, vgl. US-PS 2 491 533. Nachteilig bei diesem Verfahren ist die schwierige Herstellung von Epoxyfettalkoholen sowie der Umstand, daß mit der OH-Gruppe des Epoxyfettalkohols bei der Ringöffnung teilweise inter- und/oder intramolekulare Kondensationsprodukte gebildet werden.

Es wurde nun gefunden, daß die Titelverbindungen in besonders einfacher Weise und ohne nennenswerte Nebenreaktionen erhalten werden können, indem man vicinal hydroxy, alkoxy-bzw. hydroxy, hydroxyalkylenoxy-substituierte $C_{16}$-$C_{22}$-Fettsäureester der allgemeinen Formel IVa, IVb, IVc bzw. IVd

$R^4$(vic. OH, $OR^2$) - $COO^5$ (IVa)

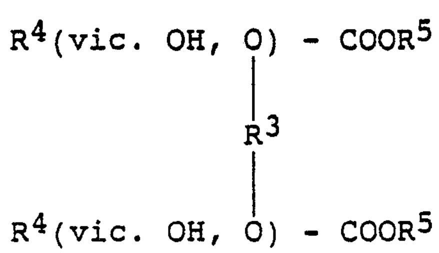

(IVb)

[$R^4$(vic. OH, $OR^2$) - COO - $]_n$ G (IVc)

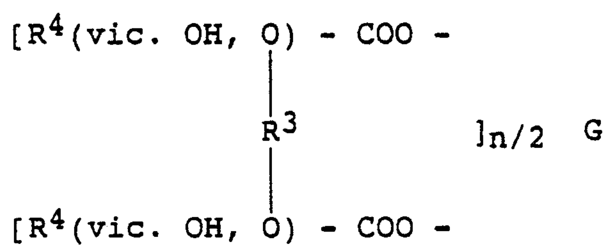

(IVd)

in denen die Gruppe $R^4$(vic. OH, $OR^2$) um eine Methylengruppe kürzer als der Rest $R^1$(vic. OH, $OR^2$) ist, $R^5$ einen Alkylrest mit 1 bis 8 Kohlenstoffatomen oder einen $C_{16}$-$C_{22}$-Alkylrest gemäß der obigen Gruppe $R^1$-(vic. OH, $OR^2$), G einen Rest eines mehrfunktionellen Alkanols mit 2 bis 3 Kohlenstoffatomen und 2 bis 3 Hydroxylgruppen bedeutet und n eine Zahl von 1 bis 3, jedoch nicht größer als die OH-Funktionalität des mehrfunktionellen Alkanols ist sowie $R^2$ und $R^3$ wie oben definiert sind, mit kupfer- und/oder zinkhaltigen Katalysatoren bei erhöhten Temperaturen und Druck hydriert.

Reste der die Gruppe $R^2$ bildenden Monoalkanole sind insbesondere die von geradkettigen oder verzweigten, gesättigten oder ungesättigten Alkanolen mit primären OH-Gruppen wie Methanol, Ethanol, Propanol, Butanol, Pentanol, Hexanol, Octanol, Decanol, Dodecanol, Tetradecanol, Hexadecanol, Oleylalkohol, Octadecanol, Behenylalkohol und Erucylalkohol einschließlich technischer Gemische derselben, wie sie in der Fettchemie üblicherweise eingesetzt werden.

Gemäß einer bevorzugten Ausführungsform des Verfahrens der Erfindung verwendet man einen Fettalkoholester der allgemeinen Formeln IVa bis IVd, in dem die Gruppe $R^2$ bzw. $R^3$ den Rest eines mehrfunktionellen Alkanols aus der von Ethylenglykol, Diethylenglykol, Propylenglykol-1,2, Propylenglykol-1,3, Butandiol-1,4, Hexandiol-1,6, Decandiol-1,10, Dodecandiol-1,12, Glycerin, Diglycerin, Trimethylolpropan, Di-trimethylolpropan und Pentaerythrit gebildeten Gruppe bedeutet.

Für das Verfahren der Erfindung können als Katalysatoren diejenigen kupfer- und/oder zinkhaltigen Katalysatoren verwendet werden, die üblicherweise für die Hydrierung von Fettsäureestern zu Fettalkoholen eingesetzt werden. Derartige Katalysatoren können neben Kupfer und/oder Zink auch weitere Metalle, z.B. Aluminium, Chrom, Vanadium, Wolfram und Molybdän sowie aktivierende Zusätze, insbesondere Barium und Cadmium, enthalten.

Bevorzugt werden in dem Verfahren der Erfindung für die Hydrierung der Fettsäureester der allgemeinen Formeln IVa bis IVd Kupferchromit-Katalysatoren verwendet, wie sie für die Direkthydrierung von Glyceridölen aus der EP-A 0 254 189 bekannt sind. Derartige Katalysatoren enthalten bevorzugt 30 bis 40 Gew.-% Cupfer, 23 bis 30 Gew.-% Chrom, 1 bis 7 Gew.-% Barium (bezogen jeweils auf oxidische Katalysatormasse) sowie gegebenenfalls weitere Übergangsmetalle, z.B. 2,5 Gew.-% Mangan, sowie gegebenenfalls 1 bis 10 Gew.-% Siliciumdioxid. Weitere Eigenschaften dieser Katalysatoren sind der EP-A 0 254 189 zu entnehmen, auf deren Inhalt hier Bezug genommen wird.

Weitere, für das Verfahren der Erfindung bevorzugte Katalysatoren sind aus der DE-B 17 68 313 bekannt, auf deren Inhalt hier Bezug genommen wird. Bei diesen Katalysatoren handelt es sich insbesondere um bariumhaltige Kupferchromit-Katalysatoren aus Bariumnitrat, Kupfernitrat und Ammoniumdichromat sowie um Kupfer-Zink-Katalysatoren aus Zinksulfat-heptahydrat und Kupfersulfat-pentahydrat.

Für das Verfahren der Erfindung geeignete Katalysatoren werden bevorzugt gepulvert in Suspension in den Ausgangsmaterialien verwendet.

Gemäß einer weiteren vorteilhaften Ausführungsform der Erfindung hydriert man die $C_{16}$-$C_{22}$-Fettsäureester der allgemeinen Formel IVa bis IVd bei Temperaturen von 200 bis 250, insbesondere 225 bis 235 °C, sowie bei Drücken von 100 bis 300 bar, insbesondere 240 bis 260 bar. Falls die Hydrierung in Gegenwart von Lösemitteln durchgeführt werden soll, eignen sich hierzu beispielsweise aliphatische Alkohole mit 1 bis 4 Kohlenstoffatomen, insbesondere diejenigen Alkohole, die auch bei der katalytischen Hydrierung entstehen.

Die Herstellung der Titelverbindungen erfolgt bevorzugt nach einem neuen Gesamtverfahren, gemäß dem man Fettsäureester der Formel Va bzw. Vb

$R^6$ - $COOR^7$ (Va)

($R^6$ - COO - $)_n$ G (Vb)

in denen $R^6$ ein $C_{15}$-$C_{21}$-Alkylenrest mit einer olefinischen Doppelbindung oder mehreren ist, $R^7$ einen Alkylrest mit 1 bis 8 Kohlenstoffatomen oder einen $C_{16}$-$C_{22}$-Alkylenrest mit einer olefinischen Doppelbindung oder mehreren bedeutet sowie G und n wie oben definiert sind, epoxidiert und die Oxirangruppen der erhaltenen Epoxyfettsäureester mit Monoalkanolen mit 1 bis 22 Kohlenstoffatomen oder mehrfunktionellen Alkanolen mit 2 bis 12 Kohlenstoffatomen und 2 bis 4 OH-Gruppen in Gegenwart von Lewissäuren aus Katalysatoren umsetzt.

Die erhaltenen Verbindungen IVa bis IVd werden dann in der oben beschriebenen Weise in die Titelverbindungen überführt.

Bevorzugt ist die Gruppe $R^6$-CO- eine lineare Alkenoylgruppe mit 16 bis 22 Kohlenstoffatomen, die eine oder mehr als eine olefinische Doppelbindung aufweisen kann, insbesondere ein Fettsäurerest, der überwiegend von Öl-, Palmitolein-, Petroselin-, Linol-, Linolen-, Gadolein-, und/oder Erucasäure abgeleitet ist. Derartige Fettsäureester sind aus synthetischen oder natürlichen Rohstoffen, z.B. aus Rindertalg, Schweineschmalz oder pflanzlichen Ölen wie Sonnenblumen-, Koriander-, Soja- oder Rapsöl, herstellbar und stellen im allgemeinen technische Gemische verschiedener Fettsäuren dar, die z.B. zusätzlich auch

Elaidin- und/oder Brassidinsäure enthalten können.

Die Alkanolkomponente $R^5O$- bzw. $R^7O$- ist von einem Monoalkanol mit 1 bis 8 Kohlenstoffatomen abgeleitet, z.B. Ethanol, Propanol oder Butanol oder 2-Ethylhexanol; bevorzugt ist Methanol. Auch Glykol- bzw. Glycerinester einschließlich Partialestern derselben gemäß der allgemeinen Formel Vc bzw. Vd der vorgenannten Fettsäuren sind in dem Verfahren der Erfindung einsetzbar.

Weiterhin kann die Gruppe $R^7$ auch ein $C_{16}$-$C_{22}$-Fettalkenylrest sein, der eine olefinische Doppelbindung oder mehrere aufweist, insbesondere eine Oleyl-, cis-6-Octadecenyl-, Palmitoleyl-, Linoleyl-, Gadoleyl-, Elaidinyl-, Linolenyl-und/oder Erucylgruppe. Aus derartigen ungesättigten Fettsäurealkylenestern können nach Epoxidation und Ringöffnung Verbindungen des Typs

$R^4$(vic. OH, $OR^2$)-CO-O-(vic. OH, $OR^2$)$R^1$

erhalten werden, die bei der Hydrierung unter gleichzeitiger Spaltung der Esterbindung 2 Moleküle Fettalkohole der Formel I ergeben.

Die Fettsäureester der Formel Va bzw. Vb können in an sich bekannter Weise epoxidiert werden, z.B. mit Persäuren, insbesondere mit in situ hergestellter Perameisensäure. Die dabei erhaltenen Epoxyfettsäureester werden mit den Monoalkanolen mit 1 bis 22 Kohlenstoffatomen oder mehrfunktionellen Alkanolen mit 2 bis 12 Kohlenstoffatomen und 2 bis 4 OH-Gruppen in Gegenwart katalytisch wirkender Lewissäuren umgesetzt; typische Beispiele für derartige Lewissäuren sind Schwefelsäure, Methansulfonsäure, Butansulfonsäure, Toluolsulfonsäure sowie saure Ionenaustauscher. Die Umsetzung erfolgt möglichst wasserfrei bei Molverhältnissen von Epoxyfettsäureestern zu mehrfunktionellen Alkanolen von 1:6 bis 2:1.

Bei Einsatzmolverhältnissen von Epoxyfettsäureestern zu mehrfunktionellen Alkanolen von etwa 2:1 entstehen durch intramolekulare Kondensation überwiegend der Einfachheit halber hier und im folgenden als "dimer" bezeichnete Fettsäureester der Formel

R (vic. OH, O) - COOR
|
$R^3$
|
R (vic. OH, O) - COOR

die als Endprodukte des Verfahrens der Erfindung die entsprechenden "dimeren" Fettalkohole der Formel I ergeben. "Dimere" Fettalkohole entstehen z.B. vorwiegend bei der Umsetzung mit Glycerin und Diglycerin, da sekundäre OH-Gruppen reaktionsträge sind.

Mit Einsatzmolverhältnissen von etwa 1:1 bis 1:6 entstehen mit mehrfunktionellen Alkanolen überwiegend "monomere" Fettsäureester bzw. als Endprodukte die "monomeren" Fettalkohole.

Die Reaktionsprodukte können nach der Umsetzung mit wasserfreien Basen, z.B. Alkali- oder Erdakalioxiden, -hydroxiden oder -carbonaten, Alkalialkoholaten, Erdalkalialkoholaten oder Aminen, neutralisiert werden; anschließend erfolgt die Entfernung des überschüssigen ein-oder mehrfunktionellen Alkanols, insbesondere durch Destillation oder Phasenverteilung.

Das Gesamtverfahren geht bevorzugt von Fettsäureestern der Formel Va bzw. Vb aus, die Jodzahlen von 55 und darüber aufweisen; im Falle mehrfach ungesättigter Fettsäureester kann man gegebenenfalls mehrere der vorhandenen olefinischen Doppelbindungen epoxidieren und kommt letzlich zu hydroxy, alkoxy- bzw. hydroxy, hydroxyalkylenoxy-substituierten $C_{16}$-$C_{22}$-Fettalkoholen, die mehr als die Mindestzahl von 2 Hydroxylgruppen aufweisen, d.h. die mehr als ein Strukturelement der Formel II enthalten.

Die Erfindung wird im folgenden anhand bevorzugter Ausführungsbeispiele näher erläutert.

In den Hydrierungsstufen der Beispiele 1 bis 6 und 8 wurde ein bariumhaltiger Kupferchromit-Kontakt analog DE-B 17 68 313 eingesetzt, der wie folgt erhalten wurde:

2,6 kg Bariumnitrat wurden in 80 l Wasser geläst und der auf 70 °C erwärmten Lösung 21,8 kg Kupfernitrattrihydrat zugefügt. Nach vollständiger Auflösung des Kupfernitrates wurde langsam und unter Rühren eine Ammoniumchromatlösung zugegeben, die durch Auflösen von 12,6 kg Ammoniumdichromat in 60 l Wasser und Zugabe von 15 l 20 %-iger Ammonialkösung hergestellt worden war. Die entstandene Suspension wurde noch 1 Stunde gerührt, der Niederschlag abfiltriert, bei 110 °C getrocknet und anschließend bei 350-450 °C geglüht. Das geglühte Produkt wurde zunächst mit insgesamt 240 l 10 %-iger Essigsäure und anschließend mit insgesamt 480 l Wasser digeriert. Das abfiltrierte Barium-Kupferchromit wurde bei 110 °C getrocknet, mit 3 Gew.-% Graphit vermischt, zunächst vorverpreßt und anschließend vermahlen.

Der im Beispiel 7 eingesetzte Kupfer/Zink-Kontakt wurde ebenfalls analog DE-B 17 68 313 erhalten:

22,5 kg Zinksulfatheptahydrat und 22,5 kg Kupfersulfatpentahydrat wurden in 160 l Wasser gelöst. Die Lösung wurde in eine zweite Lösung von 28 kg Soda in 450 l Wasser langsam unter Rühren eingegeben. Die entstandene Suspension wurde 3 Stunden bei 50 °C gerührt, anschließend filtriert und mit Wasser sulfatfrei gewaschen. Der Filterkuchen wurde bei 100 °C getrocknet und mit 3 Gew.-% Graphit vermahlen.

Beispiel 1.

Herstellung eines vicinal hydroxy, hydroxyethylenoxy-substituierten Stearylalkohols aus Ölsäuremethylester.

A. Epoxidation von Ölsäuremethylester.

Es wurde ein technischer Ölsäuremethylester mit den folgenden Kenndaten eingesetzt:

Kettenverteilung:

0 bis 0,5 % $C_{10}$
0 bis 2 % $C_{12}$
2 bis 5 % $C_{14}$
0 bis 1 % $C_{15}$
4 bis 6 % $C_{16}$ gesättigt
4 bis 6 % $C_{16}$ einfach ungesättigt
1 bis 3 % $C_{17}$
1 bis 3 % $C_{18}$ gesättigt
63 bis 73 % $C_{18}$ einfach ungesättigt
7 bis 12 % $C_{18}$ zweifach ungesättigt
0 bis 2 % $C_{18}$ dreifach ungesättigt
0 bis 2 % $C_{20}$
0 bis 2 % $C_{22}$.
Jodzahl 87,6
Verseifungszahl 193,9.

100 kg dieses Ölsäuremethylesters wurden mit 4,67 kg 85 %-iger Ameisensäure versetzt. Zu dieser Mischung wurden bei 60 °C unter Rühren und Kühlen 21,8 kg 70 %-iges Wasserstoffperoxid zugetropft. Nach Beendigung der Zugabe wurde weitere 5 Stunden bei 60 °C gerührt. Nachdem im Gaschromatogramm kein Ölsäuremethylester mehr nachweisbar war, wurde mit Wasser neutral gewaschen (pH 6 bis 7); das Epoxid wurde im Vakuum bei 90 °C getrocknet.

Analytische Daten des Epoxids:

Epoxid-O-Gehalt 4,95 %
Verseifungszahl 188,4
Jodzahl 1,7
Säurezahl 0,4.

B. Ringöffnung des epoxidierten Ölsäuremethylesters mit Ethylenglykol.

7 Mol des vorstehend erhaltenen epoxidierten Ölsäuremethylesters und 14 Mol Ethylenglykol wurden in Gegenwart von konzentrierter Schwefelsäure (0,12 g/Mol Epoxid) unter Rühren auf 90 °C erhitzt. Die anfangs exotherme Reaktion war nach 1,5 Stunden beendet (Epoxid-O: 0). Die als Katalysator eingesetzte Säure wurde mit Calciumhydroxid neutralisiert. Das Rohprodukt wurde im Vakuum bis 200 °C destilliert; das ausgefallene Calciumsulfat wurde abgesaugt. Man erhielt das Ringöffnungsprodukt in Form einer gelben Flüssigkeit mit den folgenden analytischen Daten:
Hydroxylzahl 234,3

Verseifungszahl 161,0
Jodzahl 7,1
Säurezahl 0,9.

C. Hydrierung des Ringöffnungsproduktes.

Das in Stufe B erhaltene Ringöffnungsprodukt des Stearinsäuremethylesterepoxids mit Ethylenglykol wurde mit 10 Gew.-% des Kupferchromit-Katalysators bei 230 °C und 250 bar Wasserstoffdruck im Autoklaven 4 h gerührt; der Katalysator wurde abfiltriert. Das Filtrat wurde im Ölpumpenvakuum bis 150 °C andestilliert. Man erhielt die Titelverbindung in Form einer gelben, klaren, mäßig viskosen Flüssigkeit mit den folgenden analytischen Daten:
Hydroxylzahl 435,6
Verseifungszahl 1,2
Säurezahl 0,2
Jodzahl 2,4.

Beispiel 2.

Herstellung eines vicinal hydroxy, hydroxyethylenoxy-substituierten Stearylalkohols aus Oleyloleat.

Analog zu der in Beispiel 1 beschriebenen Weise wurde zunächst aus einem technischen, handelsüblichen Oleyloleat das Bisepoxid und anschließend daraus das Ringöffnungsprodukt mit Ethylenglykol mit einer Hydroxylzahl von 238,6 und einer Verseifungszahl von 87,5 hergestellt.

Das Ringöffnungsprodukt wurde mit 10 Gew.-% des Kupferchromit-Katalysators bei 230 °C und 250 bar Wasserstoffdruck im Autoklaven 4 h gerührt. Anschließend wurde der Katalysator abfiltriert; das Filtrat wurde im Ölpumpenvakuum bis 155 °C andestilliert. Man erhielt eine klare, leicht gelbliche, mäßig viskose Flüssigkeit mit den folgenden analytischen Daten:
Hydroxylzahl 408,9
Verseifungszahl 1,7
Säurezahl 0,2
Jodzahl 1,8
Viskosität 2144 mPas.

Beispiel 3.

Herstellung eines vicinal hydroxy, hydroxyethylenoxy-substituierten Sojafettalkohols.

Aus einem handelsüblichen epoxidierten Sojaöl wurde zunächst analog zu der in Beispiel 1 beschriebenen Weise das Ringöffnungsprodukt mit Ethylenglykol (Hydroxylzahl 240,3, Verseifungszahl 157,9) hergestellt.

Die Hydrierung analog zu der in Beispiel 1 beschriebenen Weise ergab ein Produkt mit den folgenden analytischen Daten:
Hydroxylzahl 386,4
Verseifungszahl 1,3
Säurezahl 0,2
Jodzahl 2,9.

Beispiel 4.

Herstellung eines vicinal hydroxy, hydroxydiethylendioxy-substituierten Sojafettalkohols.

Analog zu der in Beispiel 1 beschriebenen Weise wurde aus einem handelsüblichen epoxidierten Sojaöl zunächst das Ringöffnungsprodukt mit Diethylenglykol mit einer Hydroxylzahl von 228,7 und einer Verseifungszahl von 143,6 hergestellt. Die Hydrierung analog zu der in Beispiel 1 beschriebenen Weise ergab die Titelverbindung mit den folgenden analytischen Daten:
Hydroxylzahl 392,1
Verseifungszahl 1,1
Säurezahl 0,4
Jodzahl 3,5.

Beispiel 5.

Herstellung eines vicinal hydroxy, hydroxypropylenoxy-substituierten Sojafettalkohols.

7 Mol eines epoxidierten Sojaöls (Epoxid-O-Gehalt 6,78 %) wurden mit 14 Mol 1,2-Propylenglykol in Gegenwart von 0,25 g/Mol konzentrierter Schwefelsäure analog zu der in Beispiel 1 beschriebenen Weise ringgeöffnet. Nach Neutralisation der Säure mit Calciumhydroxid wurde das ausgefallene Calciumsulfat abgesaugt. Das Filtrat wurde ohne Entfernung des überschüssigen Propylenglykols (zur Einstellung einer niedrigeren Viskosität) in Gegenwart des Kupferchromit-Katalysators bei 230 °C und 250 bar Wasserstoffdruck 10 h lang hydriert. Nach Andestillieren des Rohproduktes im Vakuum (0,1 mbar) bis 155 °C erhielt man die Titelverbindung in Form eines braunen, klaren Produktes mit den folgenden analytischen Daten:
Hydroxylzahl 400,7
Verseifungszahl 2,0
Säurezahl 0,2
Jodzahl 3,4.

Beispiel 6.

Herstellung eines vicinal hydroxy, hydroxyethylenoxy-substituierten Stearylalkohols.

Analog zu der in Beispiel 1 beschriebenen Weise wurde zunächst ein handelsüblicher Fettsäuremethylester mit einem Ölsäuregehalt von über 85 % (aus dem Öl einer Sonnenblumenart) epoxidiert; das Epoxid wurde anschließend mit Ethylenglykol ringgeöffnet. Das Ringöffnungsprodukt wies eine Hydroxylzahl von 193,0 und eine Verseifungszahl von 161,6 auf. Anschließend wurde das Ringöffnungsprodukt analog zu der in Beispiel 1 beschriebenen Weise, jedoch bei einer auf 6 h verlängerten Reaktionszeit, zu der Titelverbindung mit den folgenden analytischen Taten hydriert:
Hydroxylzahl 371,5
Verseifungszahl 9,6
Säurezahl 0,3
Jodzahl 3,1
Viskosität 3462 mPas.

Beispiel 7.

Herstellung eines vicinal hydroxy- hydroxyethylenoxy-substituierten Stearylalkohols mit einem Kupfer-Zink-Katalysator.

Zunächst wurde in der in Beispiel 1 beschriebenen Weise das Ringöffnungsprodukt von Stearinsäuremethylesterepoxid mit Ethylenglykol hergestellt (Hydroxylzahl 228,9; Verseifungszahl 161,4). Anschließend wurde wie in Beispiel 1 beschrieben hydriert, wobei jedoch anstelle des Kupferchromit-Katalysators der oben beschriebenen Kupfer-Zink-Kontakt verwendet wurde. Man erhielt die Titelverbindung mit den folgenden analytischen Daten:
Hydroxylzahl 432,1

Verseifungszahl 2,3
Säurezahl 0,2
Jodzahl 1,5.

Beispiel 8.

Herstellung eines "dimeren" vicinal hydroxy, hydroxyethylenoxy-substituierten Stearylalkohols.

2 Mol eines analog zu der in Beispiel 1 beschriebenen Weise erhaltenen Stearinsäuremethylesterepoxids wurden mit 1 Mol Ethylenglykol zu einem durch intramolekulare Kondensation gebildeten "dimeren" Ringöffnungsprodukt mit einer Hydroxylzahl von 101,8, einer Verseifungszahl von 172,7, einer Jodzahl von 14,8 und einer Säurezahl von 1,8 umgesetzt. Die Aufarbeitung erfolgte analog zu der für das Ringöffnungsprodukt des Beispiels 1 beschriebenen Weise. Das so erhaltene "dimere" Ringöffnungsprodukt wurde mit 10 Gew.-% des Kupferchromit-Katalysators bei 230 °C und 250 bar Wasserstoffdruck im Autoklaven 6 h gerührt. Nach Abfiltrieren des Katalysators wurde das Filtrat im Ölpumpenvakuum bis 155 °C andestilliert. Man erhielt die Titelverbindung, die einen tetrafunktionellen Alkohol darstellt, in Form eines festen, farblosen Produktes mit den folgenden analytischen Daten:
Hydroxylzahl 326,2
Verseifungszahl 4,6
Säurezahl 3,0
Jodzahl 0,2.

Beispiel 9.

Herstellung eines vicinal hydroxy, methoxy-substituierten Stearylalkohols.

500 g eines Epoxystearinsäuremethylesters, ringgeöffnet mit Methanol (Hydroxylzahl 148,4, Verseifungszahl 172,0, Säurezahl 0,3) wurde analog zu der in Beispiel 1 beschriebenen Weise 6 Stunden lang zur Titelverbindung mit den folgenden Kennzahlen hydriert:
Hydroxylzahl 343,4
Verseifungszahl 5,0
Säurezahl 0,3
Jodzahl 1,4
Gießpunkt -9 °C
Trübungspunkt <-9 °C
Viskosität ca. 500 mPas(20 °C; Höppler).

## Ansprüche

I. Verfahren zur Herstellung von Fettalkoholderivaten der allgemeinen Formel I
$R^1$(vic. OH, $OR^2$) - OH (I)
in der
$R^1$ einen vicinal disubstituierten Rest eines Fettalkohols mit 16 bis 22 Kohlenstoffatomen, der mindestens ein Strukturelement der allgemeinen Formel II
- CH(OH) - CH($OR^2$) - (II)
aufweist und
$R^2$ einen Alkylrest mit 1 bis 22 Kohlenstoffatomen, einen einbindigen Rest eines Polyols mit 2 bis 12 Kohlenstoffatomen und 2 bis 4 OH-Gruppen oder
eine Gruppe der allgemeinen Formel III

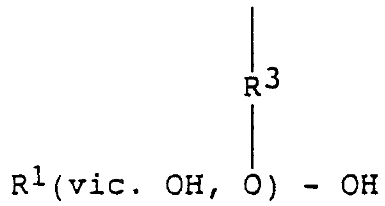

(III)

in der $R^3$ ein zweibindiger Rest eines Polyols mit 2 bis 12 Kohlenstoffatomen und 2 bis 4 OH-Gruppen, von denen mindestens 2 primäre OH-Gruppen sind, und $R^1$ wie oben definiert ist, bedeuten , dadurch gekennzeichnet, daß man einen vicinal hydroxy, alkoxy- bzw. hydroxy, hydroxyalkylenoxy-substituierten $C_{16}$-$C_{22}$-Fettsäureester der allgemeinen Formel IVa, IVb, IVc bzw. IVd

$R^4$(vic. OH, $OR^2$) - $COOR^5$ (IVa)

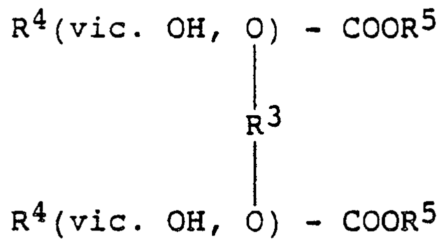

(IVb)

[$R^4$(vic. OH, $OR^2$) - COO - $]_n$ G (IVc)

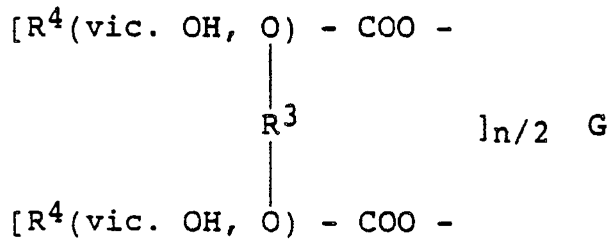

(IVd)

in denen die Gruppe $R^4$(vic. OH, $OR^2$) um eine Methylengruppe kürzer als der Rest $R^1$(vic. OH, $OR^2$) ist, $R^5$ einen Alkylrest mit 1 bis 8 Kohlenstoffatomen oder einen $C_{16}$-$C_{22}$-Alkylrest gemäß der obigen Gruppe $R^1$-(vic. OH, $OR^2$), G einen Rest eines mehrfunktionellen Alkanols mit 2 bis 3 Kohlenstoffatomen und 2 bis 3 Hydroxylgruppen bedeutet und n eine Zahl von 1 bis 3, jedoch nicht größer als die OH-Funktionalität des mehrfunktionellen Alkanols ist sowie $R^2$ und $R^3$ wie oben definiert sind, mit kupfer- und/oder zinkhaltigen Katalysatoren bei erhöhten Temperaturen und Druck hydriert.

II. Verfahren nach Anspruch 1, dadurch gekennzeichnet, daß man $C_{16}$-$C_{22}$-Fettsäureester der allgemeinen Formel IVa bis IVd verwendet, in denen die Gruppe $R^2$ bzw. $R^3$ den Rest eines mehrfunktionellen Alkanols aus der von Ethylenglykol, Diethylenglykol, Propylenglykol-1,2, Propylenglykol-1,3, Butandiol-1,4, Hexandiol-1,6, Decandiol-1,10, Dodecandiol-1,12, Glycerin, Diglycerin, Trimethylolpropan, Di-trimethylolpropan und Pentaerythrit gebildeten Gruppe bedeutet.

III.Verfahren nach Anspruch 1 oder 2, dadurch gekennzeichnet, daß man die $C_{16}$-$C_{22}$-Fettsäureester der allgemeinen Formeln IVa bis IVd an einem Kupferchromit-Katalysator oder einem Cu/Zn-Katalysator hydriert.

IV. Verfahren nach mindestens einem der Ansprüche 1 bis 3, dadurch gekennzeichnet, daß man die $C_{16}$-$C_{22}$-Fettsäureester der allgemeinen Formeln IVa bis IVd bei Temperaturen von 200 bis 250 °C, insbesondere 225 bis 235 °C, hydriert.

V. Verfahren nach mindestens einem der Ansprüche 1 bis 4, dadurch gekennzeichnet, daß man die $C_{16}$-$C_{22}$-Fettsäureester der allgemeinen Formeln IVa bis IVd bei Drücken von 100 bis 300, insbesondere 240 bis 260 bar, hydriert.

VI. Verfahren nach mindestens einem der Ansprüche 1 bis 5, dadurch gekennzeichnet, daß man die vicinal hydroxy, alkoxy- bzw. hydroxy, hydroxyalkylenoxy-substituierten $C_{16}$-$C_{22}$-Fettsäureester der allgemeinen Formeln IVa bis IVd, herstellt, indem man $C_{16}$-$C_{22}$-Fettsäureester der allgemeinen Formeln Va bzw.

Vb

$R^6$ - COOR$^7$ (Va)

($R^6$ - COO - )$_n$ G (Vb)

in denen $R^6$ ein $C_{15}$-$C_{21}$-Alkylenrest mit einer olefinischen Doppelbindung oder mehreren ist, $R^7$ einen Alkylrest mit 1 bis 8 Kohlenstoffatomen oder einen $C_{16}$-$C_{22}$ Alkylenrest mit einer olefinischen Doppelbindung oder mehreren bedeutet sowie G und n wie oben definiert sind, epoxidiert und die Oxirangruppen der erhaltenen Epoxyfettsäureester mit Monoalkanolen mit 1 bis 22 Kohlenstoffatomen oder mehrfunktionellen Alkanolen mit 2 bis 12 Kohlenstoffatomen und 2 bis 4 OH-Gruppen in Gegenwart von Lewissäuren als Katalysatoren umsetzt.

VII.Verfahren nach Anspruch 6, dadurch gekennzeichnet, daß man die Epoxyfettsäurester mit difunktionellen linearen Alkanolen mit 2 bis 12 Kohlenstoffatomen in Einsatzmolverhältnissen von etwa 2:1 umsetzt.

VIII. Verfahren nach Anspruch 6, dadurch gekennzeichnet, daß man die Epoxyfettsäureester mit den Monoalkanolen mit 1 bis 22 Kohlenstoffatomen bzw. mehrfunktionellen Alkanolen mit 2 bis 12 Kohlenstoffatomen und 2 bis 4 OH-Gruppen in Einsatzmolverhältnissen von etwa 1:1 bis 1:6 umsetzt.

Europäisches Patentamt

# EUROPÄISCHER RECHERCHENBERICHT

Nummer der Anmeldung

EP 89 11 5541

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| D,A | US-A-2 491 533 (D. SWERN)<br>* Insgesamt *<br>--- | 1-8 | C 07 C 43/13<br>C 07 C 43/11<br>C 07 C 69/708<br>C 07 D 303/40<br>C 07 C 41/26 |
| A | US-A-3 066 159 (M. DE GROOTE)<br>* Ansprüche; Tabelle I *<br>--- | 1-8 | |
| A | GB-A-1 037 021 (SWIFT)<br>* Beispiele *<br>--- | 1-8 | |
| A | "Organic reactions", vol. VIII, chapter 1, pages 1,13,18, John Wiley & Sons, Inc., New York, US; H. ADKINS: "Catalytic hydrogenation of esters to alcohols"<br>* Seiten 13,18 *<br>--- | 1-5 | |
| D,A | EP-A-0 254 189 (HENKEL)<br>* Ansprüche *<br>--- | 1-5 | |
| A | FR-A-1 600 072 (HENKEL)<br>* Zusammenfassung *<br>----- | 1-5 | |
| | | | **RECHERCHIERTE SACHGEBIETE (Int. Cl.5)** |
| | | | C 07 C 41/00<br>C 07 C 43/00<br>C 07 C 69/00 |

Der vorliegende Recherchenbericht wurde für alle Patentansprüche erstellt

| Recherchenort | Abschlußdatum der Recherche | Prüfer |
|---|---|---|
| DEN HAAG | 07-12-1989 | WRIGHT M.W. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)